# FASCICULE DE BREVET EUROPEEN

(11) **EP 3 781 089 B1**
(45) Date de publication et mention de la délivrance du brevet: **01.06.2022**
(21) Numéro de dépôt: 19703733.6
(22) Date de dépôt: 11.02.2019
(51) Int. Cl.: A61F 2/966, A61F 2/954, A61F 2/97, A61F 2/958

(54) **SYSTEME DE DEPLOIEMENT D'UNE PROTHESE VASCULAIRE POUR PONTAGE**
SYSTEM ZUR FREISETZUNG EINER BYPASS-GEFÄSSPROTHESE
SYSTEM FOR DEPLOYING A VASCULAR BYPASS PROSTHESIS

(30) Priorité: 27.02.2018 BE 201805116
(43) Date de publication de la demande: 24.02.2021
(73) Titulaire: Corquest Medtech, 1320 Beauvechain (BE)
(72) Inventeur: SEGERS, Bernard, 1050 Bruxelles (BE)
(74) Mandataire: Calysta NV
(86) Numéro de dépôt international: PCT/EP2019/053259
(87) Numéro de publication internationale: WO 2019/166208

(56) Documents cités:
- EP-A1- 0 646 365
- WO-A1-00/41632
- WO-A1-2016/022673
- WO-A2-2005/041810
- US-A1- 2003 135 257
- US-A1- 2013 060 316
- US-A1- 2014 277 361

## Description

### DOMAINE DE L'INVENTION

La présente invention se rapporte au domaine de la chirurgie vasculaire, en particulier celui du pontage artériel, par exemple entre l'aorte et les artères fémorales. La présente invention se rapporte plus particulièrement à un système de déploiement d'une prothèse vasculaire pour pontage, du type comprenant au moins un stent.

La procédure classique pour un pontage entre l'aorte et les artères fémorales est la chirurgie ouverte. Il existe donc un besoin pour une chirurgie moins invasive. Depuis les débuts récents d'un pontage aortobifémoral par laparoscopie, celle-ci a été acceptée par divers auteurs comme une alternative moins invasive dans le traitement de la maladie occlusive aortoiliaque. La laparoscopie consiste en l'examen du contenu de la cavité abdomino-pelvique au moyen d'un endoscope introduit à travers la paroi abdominale.

### ART ANTÉRIEUR

Le brevet américain n° 8.357.190 décrit un dispositif laparoscopique pour procurer un accès vasculaire, en particulier un système de déploiement pour un conduit laparoscopique comprenant un tube de greffage allongé ayant des extrémités proximale et distale. L'extrémité proximale comporte au moins deux stents auto-expansibles pour garder l'extrémité proximale ouverte, un écart étant prévu entre les deux stents pour procurer une région étanche à travers une ouverture dans une paroi du vaisseau, et une portion distale allongée non-stentée. Le système de déploiement du brevet américain n° 8.357.190 comprend des exigences techniques de construction qui en font un dispositif compliqué et coûteux. En outre ce dispositif laparoscopique, bien qu'il permette une chirurgie non-ouverte, n'a pas été à ce jour testé sur l'homme. D'après sa conception, il pourrait provoquer des saignements abondants lors du déploiement de la prothèse. Ceci est une difficulté majeure rendant cette technique impopulaire en dépit des avantages potentiels évidents pour le patient. Il existe donc un besoin pour un dispositif laparoscopique de construction plus simple, et capable de rendre cette chirurgie accessible à tous les chirurgiens vasculaires sans provoquer de saignements.

La demande de brevet US 2003/135257 décrit un système de déploiement d'une prothèse vasculaire pour pontage comprenant deux ballonnets expansibles et des moyens pour les expanser.

De même, le système de US 2014/277361, de EP 0 646 365 ou de WO 2016/022673 peut comprendre des ballonnets expansibles.

Cependant, les informations dérivables de ces publications ne permettent toujours pas de rendre cette chirurgie accessible à tous les chirurgiens vasculaires sans provoquer de saignements.

US2013060316 A1 divulgue un dispositif pour placer un stent ostial muni de deux ballonnets, l'un des ballonnets étant expansible et l'autre non.

WO0041632 A1 divulgue un dispositif de pontage utilisant un expanseur à ballonnet.

### SOMMAIRE DE L'INVENTION

Ce problème étant posé, la présente invention apporte un remède à l'anastomose aorto-prosthétique grâce au système de déploiement de la prothèse vasculaire, tel que décrit dans les revendication ci-jointes.

Le terme anastomose se réfère ici à l'ouverture d'un vaisseau sanguin en direction d'un autre, soit directement soit par connexion (suture).

A cet effet, la présente invention concerne un système de déploiement d'une prothèse vasculaire pour pontage comprenant au moins un stent, avec une portion d'extrémité à introduire dans un vaisseau et l'y relier, et comprenant une gaine agencée pour être introduite dans le vaisseau et comprenant un premier ballonnet expansible de mise en forme et stabilisation de la prothèse dans le vaisseau et un deuxième ballonnet expansible pour colmater la jonction entre ladite portion d'extrémité de la prothèse et le vaisseau, le système comprenant en outre des moyens pour expanser (autrement dit dilater) les ballonnets.

Dans le cadre d'un pontage artériel, par exemple entre l'aorte et les artères fémorales, l'utilisation d'une prothèse vasculaire comprenant au moins un stent et montée sur ballonnets expansibles est indispensable, selon la présente invention, afin d'appliquer la partie munie de stent(s) de la prothèse à l'intérieur de l'artère cible et afin de colmater la jonction entre l'artère cible et la prothèse vasculaire extra-artérielle.

Dans une forme de réalisation préférée de l'invention, les moyens pour expanser les ballonnets comprennent des premiers moyens pour expanser le premier ballonnet expansible et des deuxièmes moyens pour expanser le deuxième ballonnet expansible.

De préférence, les premiers moyens et les deuxièmes moyens d'expansion sont agencés de manière à expanser les ballonnets à des pressions différentes et/ou avec des cinétiques d'expansion différentes. Dans cette forme préférée, les ballonnets peuvent être expansés de manière indépendante l'un de l'autre, aussi bien dans le temps qu'au point de vue de la pression (facteur d'expansion / dilatation). Ceci permet d'ajuster au mieux, pendant la procédure chirurgicale, la mise en forme et la stabilisation tout en colmatant de manière efficace la jonction entre la prothèse et l'aorte sans risque de saignements.

De préférence le premier ballonnet expansible est constitué d'un matériau polymère biocompatible déformable, de préférence un élastomère, par exemple un élastomère de polyuréthanne, de latex ou de silicone, et le deuxième ballonnet expansible est constitué d'un matériau polymère biocompatible non-déformable, par exemple un polymère semi-cristallin tel que polyéthylène, poly(éthylène téréaphthalate), ou polyamide.

De préférence, la gaine est transparente et/ou déchirable, dans le sens de la longueur, à partir de son extrémité distale.

Dans une autre forme de réalisation préférée de l'invention, la prothèse vasculaire est bifurquée, afin de procurer un pontage artériel strictement anatomique. De préférence, une branche de la bifurcation est parcourue par l'ensemble comprenant les ballonnets expansibles, et l'autre branche est laissée libre.

Enfin, la partie proximale de la prothèse vasculaire peut être munie de moyens pour agripper la paroi interne du vaisseau sanguin.

Le système de déploiement de la présente invention est de construction plus simple que celui du brevet américain n° 8.357.190 déjà cité, car il ne comprend pas de moyens, tels qu'une aiguille courbée, permettant la ponction du vaisseau cible.

### DESCRIPTION D'UN MODE DE RÉALISATION PRÉFÉRÉ

La présente invention sera mieux comprise et illustrée au moyen des dessins annexés, qui cependant ne représentent qu'un mode de réalisation particulier de l'invention et ne doivent par conséquent pas être interprétés comme limitant la portée de celle-ci, laquelle est définie seulement par les revendications.

Dans les dessins annexés :
- La figure 1 représente une forme de réalisation préférée du système de déploiement de prothèse vasculaire de l'invention ;
- La figure 2 représente un système de ballonnets expansibles capable de mettre en forme et stabiliser la prothèse vasculaire dans le vaisseau et en dehors de celui-ci et de colmater la jonction entre la prothèse et le vaisseau ;
- La figure 3 représente un exemple de prothèse vasculaire bifurquée utilisable avec le système de déploiement selon l'invention et
- Les figures 4A et 4B représentent le cône d'introduction du système de déploiement selon l'invention, à deux étapes de la procédure chirurgicale, avant et après déploiement, respectivement.

Dans la description suivante :
- les termes distal et proximal doivent être entendus de manière conventionnelle dans la technique, c'est-à-dire tels que définis par exemple dans le brevet américain n° 8357190,
- le terme lumière, se rapportant à un vaisseau sanguin, désigne l'espace intérieur circonscrit par ses parois et
- le terme stent se réfère à tout fil ou tube, ou toute tige, baguette ou tringle, capable d'être inséré dans la lumière d'un vaisseau sanguin, en particulier une artère.

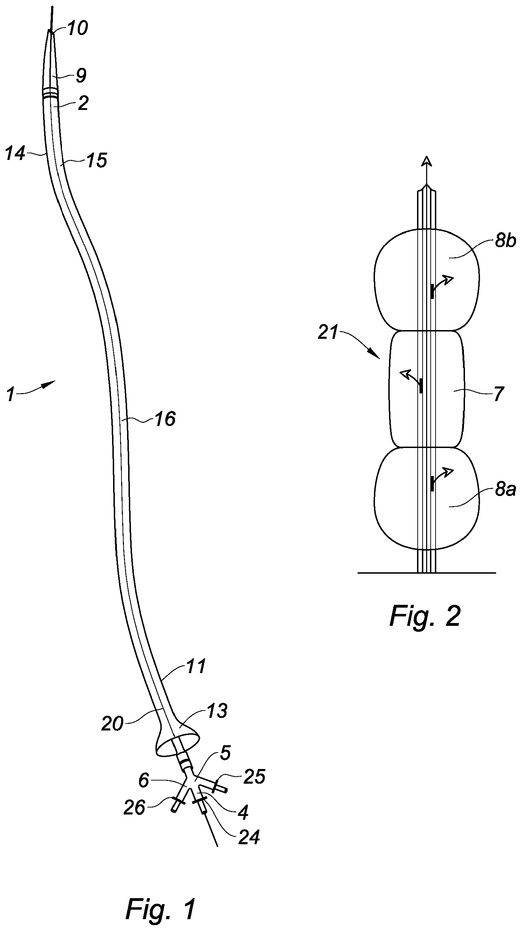

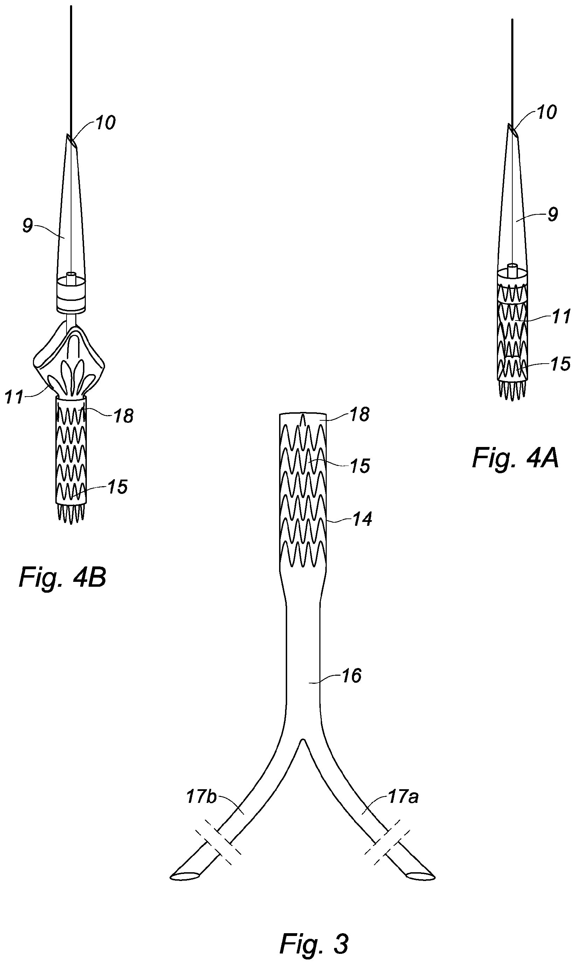

En se référant maintenant à la figure 1, on décrit un système de déploiement 1 d'une prothèse vasculaire 2, ici d'une longueur d'environ 20 à 150 cm selon les besoins de la chirurgie vasculaire envisagée. La prothèse vasculaire 2 peut être de différentes formes et de différentes longueurs, et peut notamment être bifurquée comme on le représente plus en détail sur la figure 3. La prothèse vasculaire 2 comprend d'une part, à son extrémité proximale 14, un ou plusieurs stent(s) 15 (non représenté en détail sur la figure 1) et d'autre part, à son extrémité distale, une partie restante non stentée 16. Le nombre de stents 15 dépend du patient et de la procédure chirurgicale envisagée et peut couramment atteindre 10. Par exemple, le nombre de stents 15 peut aller de 2 à 10, ou bien de 3 à 9, ou bien de 4 à 8, ou encore de 5 à 7. Les caractéristiques des stents 15, en particulier leur géométrie, leur taille et le matériau dont ils sont constitués ne sont pas des paramètres cruciaux de la présente invention et peuvent être choisis de manière conventionnelle dans la technique, en particulier à partir des modèles disponibles dans le commerce. La taille des stents 15, en particulier leur diamètre, sera choisie en fonction du vaisseau sanguin cible considéré. Chaque stent 15 mesure habituellement entre 0,5 et 3 cm de largeur. A titre d'exemple non limitatif, le diamètre distal de la prothèse vasculaire 2 peut être de l'ordre de 18 à 24 mm, et son extrémité distale mesure habituellement entre 6 et 10 cm de longueur. La prothèse vasculaire 2, avec ses différentes portions 14, 15 et 16, est destinée à être introduite dans un vaisseau sanguin tel qu'une artère, en particulier l'artère aorte. Pour cette raison, la prothèse vasculaire 2 est de préférence constituée de polytétrafluoréthylène (en abrégé PTFE, marque Teflon^{®}), poly(téréphthalate d'éthylène) (en abrégé PET, marque Dacron^{®}) ou tout autre matériau polymère biocompatible en vue de la réalisation d'un pontage artériel. Les différentes parties 14, 15, 16 de la prothèse vasculaire 2 sont incluses dans une gaine 11 elle-même agencée pour être introduite dans le vaisseau sanguin. Dans une forme de réalisation préférée, la gaine 11 est transparente afin de permettre la visualisation de la zone munie de stents 15 de la prothèse vasculaire 2 et donc de permettre un positionnement parfait de celle-ci dans l'artère. Dans une autre forme de réalisation avantageuse, l'extrémité distale 13 de la gaine 11 est déchirable dans le sens de la longueur, par exemple avec la présence d'une amorce de rupture 20, afin de faciliter son extraction lorsque le déploiement de la prothèse vasculaire 2 est achevé comme représenté sur la figure 1.

L'extrémité distale du système de déploiement comprend un ensemble 21 (figure 2) comprenant ici un ballonnet expansible 8a d'ouverture de la prothèse quand elle est positionnée dans l'aorte et destiné à parfaire le déploiement de la partie distale 16 (figure 3), un autre ballonnet expansible de colmatage 7 pour colmater la jonction entre la portion d'extrémité 14 de la prothèse et le vaisseau et surtout un ballonnet expansible 8 b de mise en forme de la prothèse destiné à l'ouvrir quand elle est dans l'aorte et à la plaquer sur l'aorte et assurer sa stabilité. Ce ballonnet 8 b a été qualifié plus haut de premier ballonnet, le ballonnet 7, de deuxième ballonnet, donc ici disposé entre les ballonnets 8a et 8b. L'extrémité proximale du système de déploiement 1 est chapeautée par un cône d'introduction 9 dont l'extrémité 10 est en forme de biseau, ou toute autre forme appropriée pour faciliter son introduction dans le vaisseau sanguin sans l'endommager. L'extrémité distale 13 de la gaine 11 a une forme évasée pour permettre ou faciliter le retrait du système après le déploiement de la prothèse vasculaire 2. A cette extrémité, le système de déploiement 1 comprend les moyens 5, 6, 25, 26 pour expanser les ballonnets de l'ensemble de ballonnets 21. A cette même extrémité, le système de déploiement 1 peut aussi comprendre un moyen 4 pour injecter du sérum physiologique, ou tout autre produit liquide utilisé en chirurgie endovasculaire, dans la lumière axiale pour assurer une lubrification intéressante.

Dans l'ensemble 21, le ballonnet expansible 7 est donc disposé entre les deux ballonnets expansibles 8a et 8b. Le nombre et les dimensions des ballonnets ne constituent pas des exigences de la présente invention, et pourront être modifiés en tenant compte de la procédure chirurgicale particulière envisagée. Quoique dans certains cas, un nombre plus élevé de ballonnets pourrait en théorie améliorer l'efficience du système, on appréciera le fait qu'en général le nombre de ballonnets n'a pas besoin d'être supérieur à 3.

Dans la forme de réalisation ici décrite, les ballonnets expansibles 8a et 8b sont constitués d'un matériau polymère biocompatible déformable. La possibilité de déformation de ce matériau polymère est telle que le premier ballonnet 8b permet d'ouvrir la prothèse (en ce compris le(s) stent(s) 15) en épousant le diamètre du vaisseau (par exemple, l'aorte) sans trop le sur-dimensionner, de manière à éviter une contrainte trop importante sur les parois du vaisseau (artère). Quant au ballonnet 8a, il assure le déploiement de la partie distale 16 non stentée de la prothèse en dehors du vaisseau. De nombreux exemples de matériaux polymères biocompatibles déformables sont connus de l'homme du métier, et commercialement disponibles, tels que, des élastomères biocompatibles. Dans une forme de réalisation de l'invention, ledit matériau polymère biocompatible déformable comprend un élastomère de polyuréthanne, le cas échéant en mélange avec un autre élastomère, ou bien est constitué essentiellement de polyuréthanne. Dans une autre forme de réalisation, ledit matériau polymère biocompatible déformable peut être un élastomère de latex ou de silicone.

Le deuxième ballonnet expansible de colmatage 7 est ici constitué d'un matériau polymère biocompatible essentiellement non-déformable. La non-déformabilité de ce matériau polymère est telle que ce deuxième ballonnet 7 permet d'appliquer de manière stable la partie munie de stent(s) 15 de la prothèse vasculaire 2 à l'intérieur du vaisseau (artère) cible, et de colmater efficacement la zone de jonction entre l'extrémité de la prothèse et le vaisseau. De nombreux exemples de matériaux polymères biocompatibles non-déformables sont connus de l'homme du métier, et commercialement disponibles, tels que, des polymères semi-cristallins biocompatibles. Des matériaux semi-cristallins ont, contrairement à des matériaux amorphes, une structure moléculaire fortement ordonnée avec des points de fusion aigus. Ils ne se ramollissent pas graduellement au fur et à mesure d'une augmentation de température, au contraire ils restent solides jusqu'à ce qu'une quantité donnée de chaleur ait été absorbée. Dans une forme de réalisation de l'invention, des exemples de tels polymères semi-cristallins comprennent le polyamide, le polyéthylène, le polypropylène, des copolymères éthylène-propylène, et des polyesters biocompatibles tels que le polyéthylène téréphthalate et des poly-hydroxyalcanoates.

En se référant maintenant à nouveau à la figure 1, on décrit maintenant plus en détails le système d'expansion des ballonnets. Les moyens d'expansion des ballonnets comprennent des sources de mise en pression, comme des conduits 5, 6, avec des robinets d'arrêt ou de passage 25,26 pour dilater, à travers les conduits 5,6, les ballonnets selon des pressions différentes et/ou selon des cinétiques d'expansion différentes. De cette manière, il est possible de faire face à des difficultés qui pourraient apparaitre au chirurgien vasculaire surveillant sur écran la procédure de déploiement, telles qu'une trop grande contrainte sur l'artère et/ou une trop grande contrainte sur la prothèse, en modulant le facteur d'expansion appliqué à chaque section. Dans une forme de réalisation préférée de l'invention, le ballonnet 7 possède un diamètre prédéterminé pour colmater la jonction de la prothèse et du vaisseau. A titre d'exemple non limitatif, une pression de 6 atmosphères convient généralement pour un diamètre de 5 mm, et une pression de 8 atmosphères pour un diamètre de 5,1 mm. La figure 1 montre ici un conduit central 4 pour, le robinet 24 étant ouvert, injecter du sérum physiologique ou tout autre produit liquide utilisé en chirurgie endovasculaire dans la lumière axiale.

En référence à la figure 3, on décrit maintenant un exemple de prothèse vasculaire 2 bifurquée adaptée au système de déploiement 1 de l'invention. Dans cette forme de réalisation préférée de l'invention, la prothèse vasculaire est bifurquée au niveau distal de sa partie non-stentée 16, afin de pouvoir envisager un pontage strictement anatomique. Par exemple, elle se bifurque au-delà de 6 à 8 cm en deux jambages (bifurcations) distincts 17a, 17b, mesurant chacun entre 15 et 40 cm de longueur, et entre 7 et 10 mm de diamètre. Le jambage 17b, qui n'est pas en rapport avec la partie munie des stents 15, est de préférence fermé à son extrémité distale, de manière à éviter des fuites lors du déploiement. Il est également possible d'agripper ce jambage avec une pince chirurgicale. La figure 3 montre aussi, à l'autre extrémité proximale de la prothèse vasculaire 2, des moyens d'agrippage 18, ici des crochets ou des agrafes chirurgicales, pour agripper la paroi interne du vaisseau.

En référence aux figures 4a et 4b, on montre de manière plus détaillée comment la portion d'extrémité proximale du système 1 comportant au moins un stent 15, située à l'extrémité proximale de la gaine 11, est munie d'un cône 9. De préférence, comme représenté sur ces figures, l'extrémité 10 du cône 9 est biseautée afin de faciliter l'introduction dans l'artère du patient sans endommager la paroi du vaisseau. A titre d'exemple non limitatif, l'extrémité distale du cône 9 a un diamètre externe de 0,8 mm et atteint progressivement, sur une distance de 2 à 5 cm, le diamètre de la gaine 11.

Toutes les dimensions (longueur, diamètre, etc.) des différents composants du système de déploiement selon la présente invention ne sont mentionnées ici qu'à titre indicatif correspondant aux cas les plus usuels, mais ne doivent pas être interprétées comme des paramètres restrictifs de l'invention.

## Revendications

1. Système (1) de déploiement d'une prothèse vasculaire (2) pour pontage comprenant au moins un stent (15), avec une portion d'extrémité (14) à introduire dans un vaisseau et l'y relier, comprenant une gaine (11) agencée pour être introduite dans le vaisseau et comprenant un premier ballonnet expansible (8b) de mise en forme et stabilisation de la prothèse dans le vaisseau et un deuxième ballonnet expansible (7) pour colmater la jonction entre ladite portion d'extrémité (14) de la prothèse et le vaisseau, le système comprenant en outre des moyens (5, 6, 25, 26) pour expanser les ballonnets (8b, 7), **caractérisé en ce que** le premier ballonnet expansible (8b) est constitué d'un matériau polymère biocompatible déformable et le deuxième ballonnet expansible (7) est constitué d'un matériau polymère biocompatible non-déformable.

2. Système (1) de déploiement d'une prothèse vasculaire (2) selon la revendication 1, dans lequel, la prothèse (2) comportant une partie distale (16), il est prévu un troisième ballonnet expansible (8a) agencé pour ouvrir la prothèse quand elle se trouve dans le vaisseau et parfaire le déploiement de ladite partie distale (16).

3. Système de déploiement (1) selon l'une des revendications 1 et 2, dans lequel il est prévu un moyen (4) pour y injecter un sérum physiologique de lubrification.

4. Système selon l'une des revendications 1 à 3, dans lequel la gaine (11) comporte, à son extrémité distale (13), une amorce de rupture (20).

5. Système de déploiement (1) selon l'une des revendications 1 à 4, dans lequel les moyens (5, 6, 25, 26) pour expanser les ballonnets comprennent des premiers moyens (5, 25) pour expanser le premier ballonnet (8b) et des deuxièmes moyens (6, 26) pour expanser le deuxième ballonnet (7).

6. Système de déploiement (1) selon la revendication 5, dans lequel les premiers moyens d'expansion (5,25) et les deuxièmes moyens d'expansion (6,26) sont agencés de manière à expanser les ballonnets à des pressions différentes et/ou avec des cinétiques d'expansion différentes.

7. Système de déploiement (1) selon l'une des revendications 1 à 6, dans lequel la prothèse vasculaire (2) est bifurquée, permettant un pontage strictement anatomique.

8. Système de déploiement (1) selon la revendication 7, dans lequel la prothèse comporte une branche (17a) parcourue par l'ensemble 21 qui comprend les ballonnets expansibles (7, 8b, 8a), et une autre branche (17b) qui est laissée libre.

9. Système de déploiement (1) selon l'une des revendications 1 à 8, dans lequel la prothèse vasculaire (2) est pourvue de moyens (18) pour agripper la paroi interne du vaisseau.

## Patentansprüche

1. System (1) zur Entfaltung einer Gefäßprothese (2) als Bypass, umfassend mindestens einen Stent (15) mit einem Endabschnitt (14) zum Einführen in ein Gefäß und Verbinden damit, umfassend eine Umhüllung (11), angeordnet, um in das Gefäß eingeführt zu werden und umfassend einen ersten expandierbaren Ballon (8b) zur Formgebung und Stabilisierung der Prothese in dem Gefäß und einen zweiten expandierbaren Ballon (7) zum Abdichten der Verbindungsstelle zwischen dem Endabschnitt (14) der Prothese und dem Gefäß, wobei das System des Weiteren Mittel (5, 6, 25, 26) zum Expandieren der Ballons (8b, 7) umfasst, **dadurch gekennzeichnet, dass** der erste expandierbare Ballon (8b) aus einem biokompatiblen, verformbaren Polymermaterial besteht und der zweite expandierbare Ballon (7) aus einem biokompatiblen, nicht verformbaren Polymermaterial besteht.

2. System (1) zur Entfaltung einer Gefäßprothese (2) nach Anspruch 1, wobei bei der ein distales Teil (16) beinhaltenden Prothese (2) ein dritter expandierbare Ballon (8a) vorgesehen ist, angeordnet zum Öffnen der Prothese, wenn sie sich in dem Gefäß befindet, und die Entfaltung des distalen Teils (16) zu vollenden.

3. System zur Entfaltung (1) nach einem der Ansprüche 1 und 2, wobei ein Mittel (4) vorgesehen ist, um dort eine physiologische Kochsalzlösung zur Schmierung zu injizieren.

4. System nach einem der Ansprüche 1 bis 3, wobei die Umhüllung (11) an ihrem distalen Ende (13) eine Sollbruchstelle (20) beinhaltet.

5. System zur Entfaltung (1) nach einem der Ansprüche 1 bis 4, wobei die Mittel (5, 6, 25, 26) zum Expandieren der Ballons erste Mittel (5, 25) zum Expandieren des ersten Ballons (8b) und zweite Mittel (6, 26) zum Expandieren des zweiten Ballons (7) umfassen.

6. System zur Entfaltung (1) nach Anspruch 5, wobei die ersten Mittel zur Expansion (5, 25) und die zweiten Mittel zur Expansion (6, 26) auf eine Weise angeordnet sind, um die Ballons bei unterschiedlichen Drücken und/oder mit unterschiedlicher Expansionskinetik zu expandieren.

7. System zur Entfaltung (1) nach einem der Ansprüche 1 bis 6, wobei die Gefäßprothese (2) gegabelt ist, was einen strikt anatomischen Bypass gestattet.

8. System zur Entfaltung (1) nach Anspruch 7, wobei die Prothese einen Ast (17a), der von dem Ensemble 21 durchlaufen wird, das die expandierbaren Ballons (7, 8b, 8a) umfasst, und einen anderen Ast (17b) beinhaltet, der frei gelassen wird.

9. System zur Entfaltung (1) nach einem der Ansprüche 1 bis 8, wobei die Gefäßprothese (2) mit Mitteln (18) zum Greifen der Innenwand des Gefäßes bereitgestellt ist.

## Claims

1. System (1) for deploying a vascular bypass prosthesis (2) comprising at least one stent (15), with an end portion (14) to be introduced into a vessel and to connect it there, comprising a sheath (11) designed to be introduced into the vessel and comprising a first expansible balloon (8b) for shaping and stabilising the prosthesis in the vessel and a second expansible balloon (7) for sealing the junction between said end portion (14) of the prosthesis and the vessel, the system further comprising means (5, 6, 25, 26) for expanding the balloons (8b, 7), **characterised in that** the first expansible balloon (8b) is formed from a deformable biocompatible polymer material and the second expansible balloon (7) is formed from a non-deformable biocompatible polymer material.

2. System (1) for deploying a vascular prosthesis (2) according to claim 1, wherein the prosthesis (2) comprising a distal portion (16), a third expansible balloon (8a) is provided and designed to open the prosthesis when it is in the vessel and accomplish the deployment of said distal portion (16).

3. System for deploying (1) according to one of claims 1 and 2, wherein a means (4) is provided for injecting therein a lubrication physiological serum.

4. System according to one of claims 1 to 3, wherein the sheath (11) comprises, at the distal end (13) thereof, an incipient break (20).

5. System for deploying (1) according to one of claims 1 to 4, wherein the means (5, 6, 25, 26) for expanding the balloons comprise first means (5, 25) for expanding the first balloon (8b) and second means (6, 26) for expanding the second balloon (7).

6. System for deploying (1) according to claim 5, wherein the first means for expanding (5, 25) and the second means for expanding (6, 26) are designed in such a way as to expand the balloons at different pressures and/or with different expansion kinetics.

7. System for deploying (1) according to one of claims 1 to 6, wherein the vascular prosthesis (2) is branched off, allowing for a strictly anatomical bypass.

8. System for deploying (1) according to claim 7, wherein the prosthesis comprises a branch (17a) travelled by the assembly 21 which comprises the expansible balloons (7, 8b, 8a), and another branch (17b) that is left free.

9. System for deploying (1) according to one of claims 1 to 8, wherein the vascular prosthesis (2) is provided with means (18) for gripping the internal wall of the vessel.
